# EUROPÄISCHE PATENTSCHRIFT

(11) **EP 3 420 902 B1**
(45) Veröffentlichungstag und Bekanntmachung des Hinweises auf die Patenterteilung: **23.08.2023**
(21) Anmeldenummer: 17178632.0
(22) Anmeldetag: 29.06.2017
(51) Int. Cl.: G06F 18/00, G06V 10/70, A61B 6/03, G06T 7/00, A61B 6/00, G16H 50/30, G06F 18/243, G06V 10/764

(54) **VERFAHREN ZUR ERMITTLUNG EINER GEWEBEVERTEILUNGSINFORMATION AUS RÖNTGENBILDDATEN, RÖNTGENEINRICHTUNG, COMPUTERPROGRAMM UND ELEKTRONISCH LESBARER DATENTRÄGER**
METHOD FOR DETERMINING TISSUE DISTRIBUTION INFORMATION MADE UP OF X-RAY IMAGING DATA, X-RAY DEVICE, COMPUTER PROGRAM AND ELECTRONICALLY READABLE DATA CARRIER
PROCÉDÉ DE DÉTERMINATION D'UNE INFORMATION DE DISTRIBUTION DE TISSU PROVENANT DE DONNÉES RADIOLOGIQUES, DISPOSITIF DE RADIOLOGIE, PROGRAMME INFORMATIQUE ET SUPPORT DE DONNÉES LISIBLE ÉLECTRONIQUEMENT

(43) Veröffentlichungstag der Anmeldung: 02.01.2019
(73) Patentinhaber: Siemens Healthcare GmbH, 91052 Erlangen (DE)
(72) Erfinder: Lu, Jing, Shanghai, 201315 (CN); Schöbinger, Max, 96114 Hirschaid (DE); Wels, Michael, 96047 Bamberg (DE)

(56) Entgegenhaltungen:
- DANIEL R. OBAID ET AL: "Dual-energy computed tomography imaging to determine atherosclerotic plaque composition: A prospective study with tissue validation", JOURNAL OF CARDIOVASCULAR COMPUTED TOMOGRAPHY, Bd. 8, Nr. 3, 1. Mai 2014 (2014-05-01), Seiten 230-237, XP055372506, AMSTERDAM, NL ISSN: 1934-5925, DOI: 10.1016/j.jcct.2014.04.007
- Anonymous: "Image Processing Fundamentals - Segmentation", , 1. September 2015 (2015-09-01), XP055436819, Gefunden im Internet: URL:https://web.archive.org/web/2015090110 3355/http://www.mif.vu.lt/atpazinimas/dip/ FIP/fip-Segmenta.html [gefunden am 2017-12-20]

## Beschreibung

Die Erfindung betrifft ein Verfahren zur Ermittlung einer Gewebeverteilungsinformation in einem interessierenden Bereich aus wenigstens einem dreidimensionalen Röntgenbilddatensatz eines Patienten. Daneben betrifft die Erfindung eine Röntgeneinrichtung, ein Computerprogramm und einen elektronisch lesbaren Datenträger.

Koronare Herzerkrankungen (Coronary Heart Disease - CHD) sind die häufigste Todesursache in den Vereinigten Staaten von Amerika mit etwa 600.000 Toten pro Jahr. Zur Diagnose von Erkrankungen der Herzkranzgefäße wurde vorgeschlagen, die Herz-Computertomographie-Angiographie (Cardiac Computed Tomography Angiography - CTA) heranzuziehen. Der Stand der Technik in der Auswertung von Röntgenbilddatensätzen des Blutgefäßsystems des Patienten im Herzbereich sieht vor, das Lumen der Koronararterien hinsichtlich deutlicher Verengung zu überprüfen. Wenn eine Verengung von mehr als beispielsweise 50 % vorliegt, wird eine Läsion als hämodynamisch relevant klassifiziert. Problematisch ist jedoch, dass in frühen Stadien der Erkrankung von Koronararterien ein Arzt entscheiden muss, ob die Läsion sich derart entwickeln wird, dass ein hohes Risiko besteht, insbesondere also akute Symptome ausgelöst werden, oder ob sie als eine zeitlich stabile Läsion aufgefasst werden kann, für die eine pharmazeutische Therapie ausreichend ist.

Ein wichtiger diagnostischer Marker, der bei dieser Entscheidung berücksichtigt wird, ist die Menge und Art von Plaque in der Blutgefäßwand, die die lokale Verengung auslöst. Koronararterien-Plaques können aus unterschiedlichen Arten von Plaque-Gewebe bestehen, welche üblicherweise in folgende Gewebearten unterteilt werden: Kalzifizierte Plaque, lipidreiche Plaque und fibrotische Plaque. Es stellt sich jedoch als äußerst kompliziert dar, eine exakte quantitative Plaque-Analyse der Gewebearten bei solchen Läsionen vorzunehmen, wenn Röntgenbilddaten mit einem bestimmten Röntgenenergiespektrum verwendet werden. Dies liegt darin begründet, dass bestimmte Gewebearten vergleichbare Schwächungswerte beziehungsweise konkret HU-Werte (Hounsfield Units) aufweisen, beispielsweise lipidreiche/nekrotische und fibrotische Plaque, während andere Gewebearten deutlich separierbar sind, beispielsweise kalzifizierte Plaque. Dies ist besonderes nachteilhaft in Fällen, in denen ein größerer lipidreicher nekrotischer Kern mit einer dünnen fibrotischen Kappe vorliegt, da eine derartige Ausgestaltung stark zur möglichen Verwundbarkeit solcher atherosklerotischer Plaque-Ansammlungen beiträgt, vgl. hierzu beispielsweise den Artikel von Virmani et al. "Lessons from sudden coronary death: a comprehensive morphological classification scheme for atherosclerotic lesions", Arterioscler. Throm. Vasc. Biol. 20 (2000), Seiten 1262 - 1275.

In einem Artikel von Daniel R. Obaid et al., "Dual-energy computed tomography imaging to determine atherosclerotic plaque composition: A prospective study with tissue validation", Journal of Cardiovascular Computed Tomography 8 (2014), Seiten 230 - 237, wurde ein anderer Ansatz vorgeschlagen, in dem Dualenergie-Scans vorliegen, welche es ermöglichen, lipidreiche nekrotische und fibrotische Gewebe deutlich besser zu unterscheiden, indem der sogenannte Dualenergie-Index (DEI) verwendet wird. Dabei können allerdings Probleme auftreten, wenn auch kalzifizierte Plaque vorliegt, nachdem hier ein Überlapp der Wertebereiche im Dualenergie-Index vorliegt.

Bekannte Verfahren zur Gewebeartenanalyse im Hinblick auf atherosklerotische Plaque, die Einzelenergie-Röntgenbilddaten nutzen, unterscheiden sich untereinander hauptsächlich im Grad der Automatisierung, vgl. hierzu beispielsweise US 7 590 270 B2, US 2012/0243764 A1, den Artikel von Damini Dey et al., "Automated 3-dimensional quantification of noncalcified and calcified coronary plaque from coronary CT angiography", Journal of Cardiovascular Computed Tomography 3 (2009), Seiten 372 - 382, sowie die Case Study von L. Hofmann, "Syngo Circulation - Siemens Scientifically Validated Cardiac CT Software", abrufbar unter https://static.healthcare.siemens.com/siemens hwemhwem ssxa websites-contextroot/wcm/idc/groups/public/@global/@imaging/@ct/documents/dow nload/mdaw/mtyw/~edisp/ct syngo circulationsiemens scientifically validated cardiac ct software-00085915.pdf .

Ähnliche Probleme zum Erzeugen verlässlicher Gewebeverteilungsinformationen bestehen auch in anderen Fällen, in denen aufgrund von Röntgenbildern Gewebearten klassifiziert werden sollen.

Der Erfindung liegt daher die Aufgabe zugrunde, eine demgegenüber verbesserte Möglichkeit zur Nutzung der Röntgenbildgebung bei der Analyse hinsichtlich unterschiedlicher Gewebearten anzugeben.

Zur Lösung dieser Aufgabe weist ein Verfahren der eingangs genannten Art erfindungsgemäß folgende Schritte auf:
- Ermitteln von wenigstens zwei Röntgenbilddatensätzen aus bei unterschiedlichen, das Röntgenenergiespektrum beschreibenden Energieparametern aufgenommenen Röntgendaten,
- Bestimmen einer auf den durch die Röntgenbilddatensätze abgedeckten Raum anzuwendenden Bildmaske zur Lokalisierung des interessierenden Bereichs,
   wonach zur Zuordnung von Gewebearten zu Voxeln im interessierenden Bereich als Gewebeverteilungsinformation:
- in einem ersten Zuordnungsschritt ein erster, wenigstens zwei Gewebegruppen mit wenigstens einer Gewebeart, von denen wenigstens eine Gewebegruppe wenigstens zwei Gewebearten umfasst, unterscheidender Klassifikator, der wenigstens einen der Röntgenbilddatensätze bezüglich eines Röntgenenergiespektrums und/oder einer linearen Kombination von Röntgenbilddaten unterschiedlichere Röntgenenergiespektren auswertet, auf den interessierenden Bereich angewandt wird, wobei aus dem Ergebnis der Anwendung des ersten Klassifikators für jede mittels wenigstens eines zweiten Klassifikators zu untersuchende Gewebegruppe eine Auswahlmaske bestimmt wird, die die Bildmaske weiter auf jene Voxel einschränkt, die einer durch den wenigstens einen zweiten Klassifikator weiter zu untersuchenden, wenigstens zwei Gewebearten enthaltenden Gewebegruppe zugeordnet sind, und
- in einem zweiten Zuordnungsschritt der wenigstens eine zweite, Gewebearten der wenigstens einen weiter zu untersuchenden, wenigstens zwei Gewebearten enthaltenden Gewebegruppe unterscheidender Klassifikator, der wenigstens einen aus wenigstens zwei der Röntgenbilddatensätze ermittelten, eine nichtlineare Kombination von Röntgenbilddaten unterschiedlicher Röntgenenergiespektren enthaltenden Multienergiewert auswertet, auf den als der entsprechenden Gewebegruppe zugehörig klassifizierten Anteil des interessierenden Bereichs angewandt wird.

Die Erfindung schlägt also eine Art Hybrid-Workflow für die quantitative Gewebearten-Analyse von Mehrenergie-Röntgenbilddaten vor, welcher die Vorteile der Ein-Energiebildgebung und der Mehrenergiebildgebung gemeinsam in einem neuartigen Zugang nutzt. Der Energieparameter ist hierbei als ein Aufnahmeparameter zu verstehen, der beeinflusst, welches Energiespektrum die im Röntgenstrahler der verwendeten Röntgeneinrichtung erzeugten Röntgenstrahlen aufweisen. Üblicherweise wird zur Angabe der Energie der Röntgenstrahlen hierbei als Energieparameter die Röhrenspannung verwendet, so dass beispielsweise bei einer Dualenergiebildgebung 100 kV und 140 kV als Energieparameter verwendet werden können, um Röntgenstrahlen unterschiedlicher spektraler Verteilung der Energie, also unterschiedlichen Röntgenenergiespektrums, zu erhalten.

Erfindungsgemäß wird nun ausgenutzt, dass sich manche Gewebearten bereits deutlich in Röntgenbilddaten unterscheiden, die mit einem festen, bestimmten Röntgenenergiespektrum aufgenommen wurden. Diese Gewebearten unterscheiden sich also grundsätzlich darin, wie stark sie die Röntgenstrahlung schwächen. Schwächungseigenschaften können sich jedoch abhängig von der Energie der Röntgenstrahlen auch deutlich verändern, so dass es mithin möglich ist, durch Kombination von Röntgenbilddaten, die bei unterschiedlichen Röntgenenergiespektren gewonnen wurden, weitere Informationen abzuleiten und Gewebearten beziehungsweise Gruppen von Gewebearten zu differenzieren. Beispielsweise können Gewebearten, deren Röntgenschwächung keine oder nur geringe Energieabhängigkeit zeigt, mit Hilfe geeigneter Energiewerte von Gewebearten unterschieden werden, die eine starke Energieabhängigkeit ihrer Schwächungseigenschaften aufweisen. Die Erfindung nutzt dies aus, indem zunächst Gruppen von Gewebearten voneinander unterschieden werden, die grundsätzlich unterscheidbare Schwächungseigenschaften auch bereits bei Verwendung eines Energiespektrums aufweisen, indem ein erster Klassifikator auf entweder einen der dreidimensionalen Röntgenbilddatensätze angewandt wird oder aber auf einen durch lineare Kombination von Röntgenbilddaten unterschiedlicher Röntgenenergiespektren gewonnenen Mischbilddatensatz. Innerhalb der Gruppen von Gewebearten, wenn diese mehrere Gewebearten enthalten, kann dann ein zweiter Klassifikator eingesetzt werden, der nun eine nicht lineare Kombination von Röntgenbilddaten als Multienergiewert auswertet, um wiederum eine Unterscheidung zu ermöglichen, in diesem Fall hinsichtlich der Energieabhängigkeit. Dabei sei noch darauf hingewiesen, dass die Anwendung der Klassifikatoren selbstverständlich maximal auf den interessierenden Bereich beschränkt erfolgt, welcher mit besonderem Vorteil so gewählt ist, dass er lediglich die hier erfassten Gewebearten oder durch die Klassifikatoren deutlich ausschließbare Gewebearten enthält. Das Ergebnis der Klassifikatoren, also die Zuordnung von Gewebearten zu Voxeln, bildet die Gewebeverteilungsinformation.

Bei den Röntgenbilddatensätzen handelt es sich hierbei um dreidimensionale Röntgenbilddatensätze, die insbesondere durch Rekonstruktion aus Projektionsbildern unterschiedlicher Aufnahmegeometrien als Röntgendaten hergeleitet wurden. Entsprechende Rekonstruktionsverfahren, beispielsweise die gefilterte Rückprojektion, sind im Stand der Technik bereits bekannt, und liefern als Ergebnis lokale Schwächungswerte, üblicherweise ausgedrückt als HU-Werte (Hounsfield Units) in den Röntgenbilddaten.

Dabei können die Röntgenbilddatensätze insbesondere Röntgenbilddatensätze der Multienergie-Computertomographie, insbesondere der Dualenergie-Computertomographie, sein. Insbesondere wurden in diesem Zusammenhang bereits als Röntgeneinrichtungen spezielle Dualenergie-Computertomographie-Einrichtungen vorgeschlagen, welche mittels unterschiedlicher Röntgenstrahler, die gleichzeitig winkelversetzt bewegt werden können, unterschiedliche Röntgenenergiespektren erzeugen. Ein besonders vorteilhaftes Ausführungsbeispiel ergibt sich, wenn als interessierender Bereich ein eine Koronarplaque enthaltender Gefäßabschnitt eines Blutgefäßsystems des Patienten verwendet wird, wobei eine erste Gewebegruppe als einzige Gewebeart kalzifizierte Plaque und eine zweite Gewebegruppe als Gewebearten lipidreiche Plaque und fibrotische Plaque umfasst. Auch wenn die vorliegende Erfindung selbstverständlich auch auf die Gewebeartenunterscheidung beziehungsweise Trennung in anderen Anwendungsfällen, in denen entsprechende Eigenschaftsunterschiede hinsichtlich der Einenergie- und Mehrenergiebildgebung existieren, angewendet werden kann, stellt dieses Ausführungsbeispiel doch ein äußerst vorteilhaftes Ausführungsbeispiel dar, so dass sich viele der folgenden Ausführungen auf dieses Ausführungsbeispiel beziehen werden, ohne dass die Allgemeinheit der hiesigen Beschreibung hierdurch beeinträchtigt werden soll.

Besonders zweckmäßig kann die vorliegende Erfindung also Dualenergie- beziehungsweise Multienergie-CTA-Daten in Form der Röntgenbilddatensätze einsetzen, um zunächst in einem ersten Schritt bei Auswertung eines Plaque-Bereichs einer Koronararterie kalzifizierte Plaque von Plaque niedrigerer Schwächung, also lipidreicher Plaque und fibrotischer Plaque, zu unterscheiden, basierend auf den HU-Werten. In einem zweiten Schritt wird die Auswertung dann auf die verbleibenden Bereiche beschränkt, die nicht kalzifizierte Plaque enthalten und eine weitere Trennung zwischen lipidreicher Plaque und fibrotischer Plaque wird durch den Multienergiewert, insbesondere einen Dualenergiewert, erreicht. Mithin bietet die vorliegende Erfindung in diesem Kontext einen Workflow, der unmittelbar bestätigte beziehungsweise bekannte Schwächungscharakteristiken bezüglich atherosklerotischer Plaque sowohl im Einenergie- als auch im Multienergiebereich auf kombinierte Art und Weise nutzt. Hierbei kann der Grad an Automatisierung und Interaktion vergleichbar zu bekannten Einenergie-Plaqueanalysetools gewählt werden, wobei jedoch zusätzlich Multienergie-Röntgenbilddaten genutzt werden.

Eine zweckmäßige Weiterbildung der Erfindung sieht vor, dass die Röntgenbilddatensätze insbesondere elastisch registriert werden oder sind. Falls also nicht eine Registrierung der dreidimensionalen Röntgenbilddatensätze inhärent bereits gegeben ist, beispielsweise aufgrund der Verwendung einer Dualenergie-Computertomographie-Einrichtung, kann eine vollständig automatische, bevorzugt elastische Registrierung zu Beginn des Verfahrens erfolgen.

Wie bereits angedeutet wurde, ist es ferner zweckmäßig, wenn der erste Klassifikator einen aus den Röntgenbilddatensätzen durch Linearkombination von Röntgenbilddaten jeweiliger Voxel ermittelten Mischbilddatensatz auswertet. Beispielsweise kann hier eine lineare Kombination vorgenommen werden, die beispielsweise parametriert, wie stark welcher der dreidimensionalen Röntgenbilddatensätze in den resultierenden dreidimensionalen Röntgenbilddatensatz eingeht, indem beispielsweise eine voxelweise Addition erfolgt, bei der die Summe der Gewichtungsparameter zweckmäßigerweise 1 ergibt. Analog kann im Rahmen der vorliegenden Erfindung zweckmäßigerweise vorgesehen sein, dass der wenigstens eine Multienergiewert für die verschiedenen Voxel in einem Multienergiedatensatz des interessierenden Bereichs ermittelt wird. Sollten mehrere Multienergiewerte verwendet werden, können die Multienergiedaten des Multienergiedatensatzes für jedes Voxel selbstverständlich auch einen Vektor umfassen, der aus den mehreren Multienergiewerten gebildet wird. Das Mischbild, insbesondere wenn die Gewichtungsparameter für die einzelnen Röntgenbilddatensätze in ihrer Summe gleich eins ergeben, entspricht letztlich wiederum Schwächungswerten, mithin HU-Werten, wie ein regulärer Einenergie-CT-Scan.

Vorzugsweise wird als einer des wenigstens einen Multienergiewerts der Dualenergieindex (DEI) ermittelt. Der Dualenergieindex ist eine Größe, die auch bereits in den bereits zitierten Artikel von D. Obaid et al. eingesetzt wird. Auch in einem Beitrag zur Zwei-Quellen-Computertomographie von B. Krauss et al. in: T.R.C. Johnson et al., "Dual Energy CT in Clinical Practice", Medical Radiology, DOI: 10.1007/174_2010_44, Springer-Verlag, 2011, wurde diese Multienergiewertgröße bereits als zweckmäßig empfohlen. Selbstverständlich ist es auch denkbar, andere Multienergiewerte einzusetzen, wenn sich diese als zweckmäßig im speziellen Anwendungsfall erweisen, wie dies für den Dualenergieindex bei der Unterscheidung von Gewebearten der atherosklerotischen Plaque gilt. Beispiele für weitere Alternativen oder zusätzlich einsetzbare Multienergiewerte sind das Dualenergieverhältnis (DER - Dual Energy Ratio) sowie sonstige, insbesondere multiplikative Komponenten enthaltende Kenngrößen.

In vorteilhafter Ausgestaltung der vorliegenden Erfindung kann vorgesehen sein, dass zur Ermittlung des interessierenden Bereichs in den Röntgenbilddatensätzen eine automatische anatomische Segmentierung, insbesondere eine Segmentierung eines Blutgefäßsystems, durchgeführt wird, auf deren Grundlage der interessierende Bereich lokalisiert wird. Im Stand der Technik wurde bereits eine Vielzahl von Möglichkeiten zur automatischen Segmentierung von anatomischen Strukturen, insbesondere Blutgefäßbäumen, vorgeschlagen. Im Anwendungsfall des koronaren Blutgefäßbaums sind beispielsweise bereits eine Vielzahl von Verfahren bekannt, um beispielsweise Centerlines (Mittellinien) der Blutgefäße vollständig automatisch zu detektieren, was insbesondere auch innerhalb eines Mischbilddatensatzes, falls ein solcher ermittelt wird, erfolgen kann. Hierzu sei rein beispielhaft auf den Artikel von Zheng et al., "Model-driven centerline extraction for severely occluded major coronary arteries" in Machine Learning in Medical Imaging, LNCS, Vol. 7588 (2012), Seiten 10 - 18, verwiesen. Das Ergebnis einer solchen Segmentierung kann zweckmäßigerweise, beispielsweise in einer graphischen Nutzerschnittstelle (GUI), an einen Benutzer ausgegeben werden, die auch Mittel bereitstellen kann, existierende Segmentierungen, beispielsweise ermittelte Centerlines, zu korrigieren und insbesondere semiautomatisch eigene Centerlines zu definieren.

Was die letztendliche Lokalisierung des interessierenden Bereichs angeht, kann vorgesehen sein, dass der interessierende Bereich manuell durch einen Benutzer in einer aus den Segmentierungsergebnissen abgeleiteten Modelldarstellung der Anatomie lokalisiert wird. Zusätzlich oder alternativ kann jedoch auch eine automatische Detektion des interessierenden Bereichs unter Berücksichtigung der Segmentierung und wenigstens einer in den Röntgenbilddaten sichtbaren anatomischen Eigenschaft des interessierenden Bereichs erfolgen. Ein interaktives Verfahren, das es den Benutzer erlaubt, durch geometrische Modellierung einen gesamten Plaque-Bereich von seinem Anfang bis zu seinem Ende durch semi-automatische Markierung äußerer Gefäßwände zu definieren, ist beispielsweise aus Kretschmer et al. "Interactive Patient-Specific Vascular Modeling with Sweep Surfaces", IEEE Transactions on Visualization and Computer Graphics, Volume 19, Issue 12, Seiten 2828 - 2837, Dezember 2013, in Verbindung mit Grosskopf et al.: "Accurate, fast, and robust vessel contour segmentation of CTA using an adaptive self-learning edge model", Proc. SPIE 7259, Medical Imaging 2009: Image Processing, 2009, bekannt. Zusätzlich oder alternativ können Gefäßabschnitte, die von Plaque betroffen sind, vollständig automatisch durch geeignete algorithmische Lösungen lokalisiert werden, wie dies beispielsweise durch Keim et al. in "Detection, Grading and Classification of Coronary Stenoses in Computed Tomography Angiography", Proc. MICCAI, LNCS, Vol. 6893 (2011), Seiten 25 - 32, beschrieben ist. Hier kann wiederum seitens des Benutzers eine manuelle Korrektur erfolgen, wenn dieser mit der Wahl des interessierenden Bereichs nicht vollständig zufrieden ist.

Sowohl bei manueller als auch bei automatischer Lokalisierung des interessierenden Bereichs wird zur Lokalisierung eine auf den durch die Röntgenbilddatensätze abgedeckten Raum anzuwendende Bildmaske bestimmt. Das Ergebnis ist also ein dreidimensionales Maskenbildvolumen, welches exakt festlegt, welche Bildvoxel durch den ersten Klassifikator untersucht werden soll. Die Bildmaske kann sich bei entsprechender Ausgestaltung des Verfahrens zweckmäßigerweise auf den Mischbilddatensatz (und ggf. den Multienergiedatensatz) beziehen. Insbesondere kann sich, allgemein gesagt, die Bildmaske aus der Segmentierung heraus bereits auf ein bestimmtes Organ, insbesondere einen Blutgefäßabschnitt, beziehen, mithin auf diesen beschränkt sein, wobei es durchaus auch denkbar ist, äußere, nicht zu analysierende Anteile von der Bildmaske auszunehmen, so dass tatsächlich nur Plaquebereiche weiter betrachtet werden. Eine besonders vorteilhafte Ausgestaltung in diesem Kontext sieht vor, dass bei einem auf ein Gefäßorgan bezogenen interessierenden Bereich das Lumen des Gefäßorgans segmentiert und von der Bildmaske ausgenommen wird. Das bedeutet, eine Ausgestaltung der vorliegenden Erfindung kann vorsehen, dass das Lumen des Gefäßorgans, insbesondere des Blutgefäßabschnitts, ebenso durch die Bildmaske der Betrachtung durch die Klassifikatoren entzogen wird, was beispielsweise durch eine manuelle und/oder automatische Segmentierung der inneren Begrenzung des Gefäßorgans erfolgen kann. Auf diese Art wird bei der Betrachtung von atherosklerotischen Plaques die Trennung zwischen kalzifizierten Plaques und kontrastverstärktem Blut im Gefäßlumen verbessert, insbesondere verglichen mit einfacher HU-basierter Schwellwertbildung.

Ferner wird aus der Bildmaske und dem Ergebnis der Anwendung des ersten Klassifikators für jede mittels eines zweiten Klassifikators zu untersuchende Gewebegruppe eine Auswahlmaske bestimmt, die die Bildmaske weiter auf jene Voxel einschränkt, die auch der durch den zweiten Klassifikator weiter zu untersuchenden Gewebegruppe zugeordnet sind.

Der erste und/oder der zweite Klassifikator können wenigstens einen Schwellwertvergleich umfassen. Es kann mithin vorgesehen sein, dass für den ersten Klassifikator ein Schwellwert, insbesondere ein HU-Schwellwert, vorgegeben wird, um die Gewebegruppen voneinander trennen zu können, indem ein einfacher Vergleich von Voxelwerten wenigstens eines Röntgenbilddatensatzes beziehungsweise bevorzugt eines Mischbilddatensatzes vorgenommen wird. Entsprechend können Schwellwerte, insbesondere DEI-Schwellwerte, für den zweiten Klassifikator verwendet werden, um eine einfache Umsetzung zu erlauben. Selbstverständlich ist es jedoch auch denkbar, komplexere Methoden einzusetzen, die bezüglich des ersten Klassifikators Einenergie-Informationen nutzen und danach bezüglich des zweiten Klassifikators die zusätzliche Multienergie-Information einsetzen; dies kann insbesondere in Abhängigkeit von Bildcharakteristiken und Aufnahmeparametern bezüglich der Akquisitions-Modalität, also der Röntgeneinrichtung, die die Röntgenbilddatensätze aufnimmt, erfolgen.

Konkret kann vorgesehen sein, dass wenigstens ein den ersten und/oder den zweiten Klassifikator parametrierender Klassifikatorparameter, insbesondere ein Schwellwert, wenigstens teilweise manuell und/oder wenigstens teilweise automatisch festgelegt wird. Es kann also zum einen vorgesehen sein, dass ein Benutzer zunächst einen HU-Schwellwert für den ersten Klassifikator vorgibt, dann einen DEI-Schwellwert für den zweiten Klassifikator vorgibt. Hierbei kann beispielsweise ein Schieberegler oder dergleichen an einer graphischen Benutzerschnittstelle eingesetzt werden. Vorzugsweise kann jedoch auch die Klassifizierung, mithin die Aufteilung nach Gewebearten, ebenso weitgehend automatisiert werden, indem entsprechendes Hintergrundwissen genutzt wird.

So sieht eine bevorzugte Ausgestaltung der vorliegenden Erfindung vor, dass zur wenigstens teilweise automatischen Ermittlung wenigstens eines Klassifikatorparameters wenigstens ein Aufnahmeparameter und/oder wenigstens ein die Röntgenbilddaten beschreibender Röntgenbilddatensatzparameter ausgewertet werden und/oder ein, insbesondere durch maschinelles Lernen anhand von bezüglich der Gewebearten annotierten Trainingsdaten trainiertes, physikalisches Parametermodell, insbesondere der künstlichen Intelligenz, zur Ermittlung wenigstens teilweise automatisch zu bestimmender Klassifikatorparameter verwendet wird. Röntgenbilder unterscheiden sich zwar grundsätzlich zwischen unterschiedlichen Patienten und/oder bei unterschiedlichen Aufnahmeparametern, wobei jedoch in vielen Fällen Zusammenhänge zwischen diesen Aufnahmeparametern/Bildeigenschaften und Klassifikatorparametern bestehen, mithin hergeleitet und genutzt werden können. Beispielsweise kann in Abhängigkeit von Aufnahmeparametern und/oder Bildeigenschaften, beispielsweise in Abhängigkeit des Energieparameters beziehungsweise eines effektiven Energieparameters für einen Mischbilddatensatz als Aufnahmeparameter, ein HU-Schwellwert beziehungsweise in Abhängigkeit von den Energieparametern für alle relevanten Röntgenbilddatensätze ein DEI-Schwellwert abgeleitet werden.

Mit besonderem Vorteil lassen sich im Rahmen der vorliegenden Erfindung, insbesondere bezüglich der Automatisierung der Klassifikator-Parametrierung, Methoden der künstlichen Intelligenz und des maschinellen Lernens einsetzen. Grundlage sind hier annotierte Trainingsdaten, mithin im Beispiel der Anwendung bei atherosklerotischer Plaque dreidimensionale Röntgenbilddatensätze beziehungsweise interessierende Bereiche, bei denen einzelnen Voxeln bereits die Gewebeart zugeordnet ist. Hieraus lassen sich durch maschinelles Lernen Zusammenhänge in Parametermodellen abbilden, beispielsweise neuronalen Netzen und dergleichen, wobei als Ergebnis der Trainingsphase das Parametermodell parametriert ist und genutzt werden kann, um Eingangsdaten, beispielsweise Aufnahmeparametern und/oder Bildeigenschaften, Klassifikatorparameter, beispielsweise Schwellwerte, zuzuordnen.

Denkbar ist in diesem Zusammenhang auch eine Ausgestaltung, in der automatisch ein Vorschlag für Klassifikatiorparameter bestimmt wird, der dann von einem Benutzer, beispielsweise mit einem Schieberegler eines GUI oder dergleichen, noch feinjustiert werden kann.

Als Gewebeinformation wird in jedem Fall wenigstens die Zuordnung von Gewebearten zu Voxeln bestimmt, wobei durch weitere Auswertung auch weitere, insbesondere quantitative, Gewebeinformationen abgeleitet werden. Beispielsweise können durch Zählen von Gewebearten zugeordneten Voxeln Anteile der Gewebearten quantifiziert werden und dergleichen. Die wenigstens eine ermittelte Gewebeinformation kann auf unterschiedliche, grundsätzlich bekannte Arten ausgegeben werden. Vorteilhaft ist insbesondere die Erzeugung einer graphischen Darstellung, in der beispielsweise verschiedene Gewebearten unterschiedlich, beispielsweise in unterschiedlichen Farben, dargestellt werden können.

Neben dem Verfahren betrifft die vorliegende Erfindung auch eine Röntgeneinrichtung, die eine zur Durchführung des erfindungsgemäßen Verfahrens ausgebildete Steuereinrichtung aufweist. Bevorzugt handelt es sich bei der Röntgeneinrichtung um eine Multienergie-Computertomographie-Einrichtung. Insbesondere sind hier Dualenergie-Computertomographen zu nennen. In der erfindungsgemäßen Ausgestaltung kann unmittelbar an der Röntgeneinrichtung selbst bereits die Auswertung der Röntgenbilddatensätze hinsichtlich der Gewebearten erfolgen, indem mittels des erfindungsgemäßen Verfahrens Voxeln Gewebearten zugeordnet werden können. Sämtliche Ausführungen bezüglich des erfindungsgemäßen Verfahrens lassen sich analog auf die erfindungsgemäße Röntgeneinrichtung übertragen, mit welcher mithin die bereits genannten Vorteile ebenso erhalten werden können.

Insbesondere kann die Steuereinrichtung der Röntgeneinrichtung mithin neben den typischen, die Aufnahme von Röntgendaten steuernden Aufnahmeeinheiten auch eine Ermittlungseinheit, insbesondere Rekonstruktionseinheit, zur Ermittlung der dreidimensionalen Röntgenbilddatensätze umfassen. Ferner sind eine erste Klassifikatoreinheit und eine zweite Klassifikatoreinheit zur Anwendung der entsprechenden Klassifikatoren vorgesehen. Optional kann die Röntgeneinrichtung zusätzlich eine Registrierungseinheit und/oder eine Segmentierungseinheit und/oder eine Lokalisierungseinheit zur Lokalisierung des interessierenden Bereichs und/oder eine Interaktionseinheit zur Bedienerinteraktion aufweisen.

Ein erfindungsgemäßes Computerprogramm ist beispielsweise in den Speicher einer Recheneinrichtung, insbesondere einer Steuereinrichtung einer Röntgeneinrichtung, ladbar und weist Programmmittel auf, um die Schritte eines erfindungsgemäßen Verfahrens durchzuführen, wenn das Computerprogramm auf der Recheneinrichtung ausgeführt wird. Das Computerprogramm kann auf einem erfindungsgemäßen elektronisch lesbaren Datenträger gespeichert sein, der mithin insbesondere Steuerinformationen umfasst, die bei Verwendung des Datenträgers in einer Recheneinrichtung, insbesondere einer Steuereinrichtung einer Röntgeneinrichtung, ein erfindungsgemäßes Computerprogramm realisieren und somit den Ablauf des erfindungsgemäßen Verfahrens auf der Recheneinrichtung ermöglichen. Bei dem elektronisch lesbaren Datenträger kann es sich insbesondere um einen nicht-transienten Datenträger, insbesondere eine CD-ROM, handeln.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: einen Ablaufplan eines Ausführungsbeispiels des erfindungsgemäßen Verfahrens,
- Fig. 2: einen Graphen zur Trennung von Gewebearten in einem Röntgenbilddatensatz eines ersten Röntgenenergiespektrums,
- Fig. 3: einen Graphen zur Trennung von Gewebearten in einem Röntgenbilddatensatz eines zweiten Röntgenenergiespektrums,
- Fig. 4: einen Graphen zur Trennung von Gewebearten bezüglich eines Dualenergie-Index,
- Fig. 5: eine schematische Skizze einer Koronararterie mit atherosklerotischer Plaque,
- Fig. 6: eine mögliche Darstellung von Gewebeverteilungsinformation, und
- Fig. 7: eine erfindungsgemäße Röntgeneinrichtung.

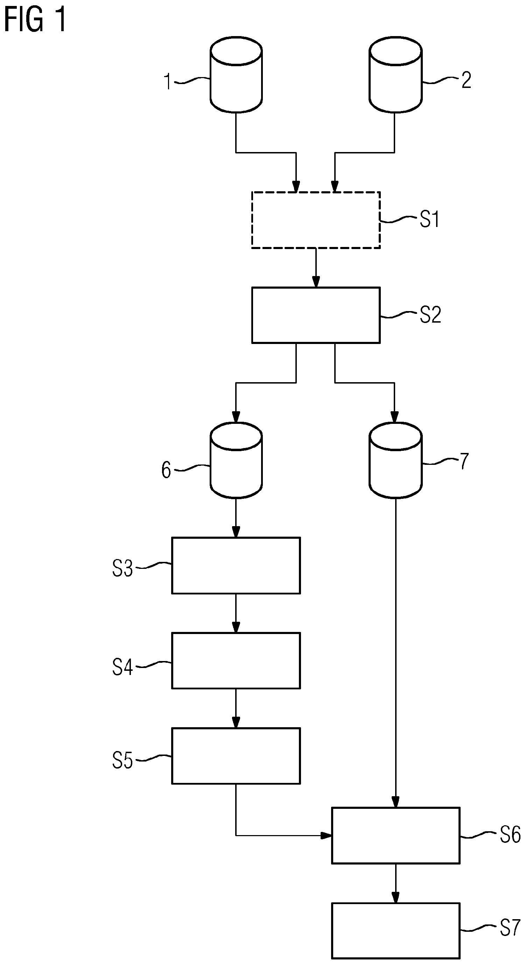

Im Folgenden wird ein Ausführungsbeispiel der vorliegenden Erfindung zur Ermittlung einer Gewebeverteilungsinformation bei einer Gefäßverengung einer Koronararterie mit Hilfe von Fig. 1 näher erläutert. Dabei wird von einer sogenannten Dualenergie-Aufnahme ausgegangen, das bedeutet, es existieren zwei Röntgenbilddatensätze 1, 2, die bei unterschiedlichen, durch einen Energieparameter beschriebenen Röntgenenergiespektren, also unterschiedlichen Spektralverteilungen der Energie der Röntgenstrahlen, aufgenommen wurden. Der Energieparameter ist vorliegend die Röhrenspannung, wobei diese zur Aufnahme des ersten Röntgenbilddatensatzes 1 (beziehungsweise der zugrundeliegenden Projektionsbilder als Röntgendaten) als 100 KV, für den zweiten Röntgenbilddatensatz als 140 KV gewählt wurde.

Bei den Röntgenbilddatensätzen 1, 2 handelt es sich naturgemäß bei einer Untersuchung der Koronararterie um angiographische Aufnahmen, konkret Aufnahmen der koronaren Computertomographie-Angiographie (CTA). Als Röntgeneinrichtung wurde dabei eine Dualenergie-Comutertomographie-Einrichtung verwendet.

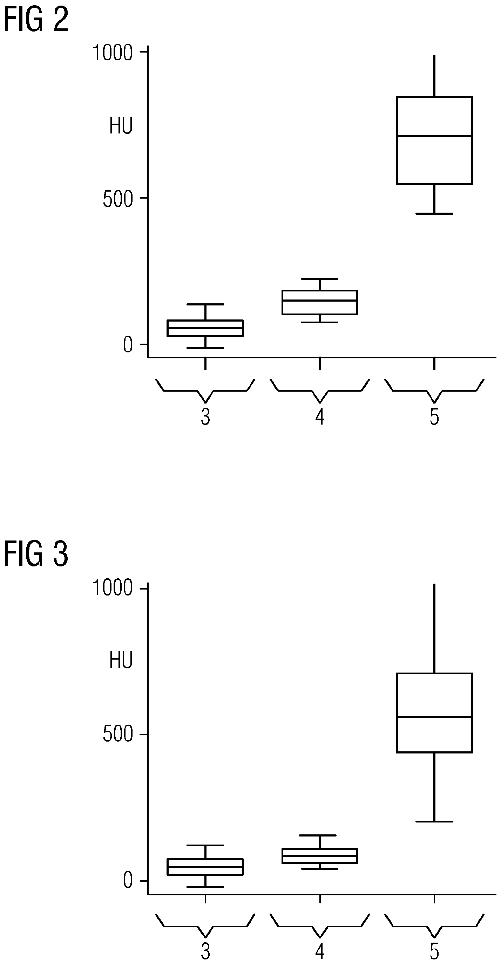

Das vorliegende Verfahren soll es ermöglichen, bei einer Gefäßverengung als Gewebearten lipidreiche Plaque, fibrotische Plaque und kalzifizierte Plaque zu unterscheiden. Die Grundlage hierzu erläutern die Figuren 2 bis 4. Figur 2 zeigt typische Schwächungswerte (HU-Werte) für unterschiedliche dieser Gewebearten mit Abweichungen, nämlich im Bereich 3 für lipidreiche Plaque, im Bereich 4 für fibrotische Plaque und im Bereich 5 für kalzifizierte Plaque. Dabei bezieht sich die Darstellung auf den ersten Röntgenbilddatensatz 1, der bei einer Röhrenspannung von 100 kV aufgenommen wurde. Ersichtlich lassen sich lipidreiche Plaque und fibrotische Plaque nicht hinreichend deutlich voneinander trennen, jedoch kann klar zwischen den Plaques niedriger Schwächung und der kalzifizierten Plaque unterschieden werden.

Ein ähnliches Bild ergibt sich im Hinblick auf Fig. 3, die sich auf eine Röhrenspannung von 140 KV bezieht.

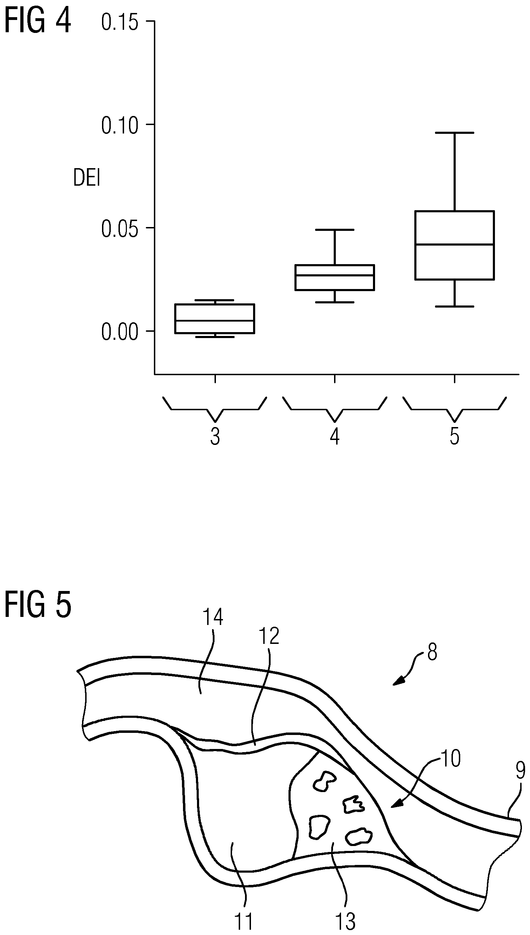

Hingegen zeigt Fig. 4 typische Werte für einen Multienergiewert, hier den Dualenergieindex (DEI), wie er aus den Röntgenbilddaten des ersten Röntgenbilddatensatzes 1 und des zweiten Röntgenbilddatensatzes 2 ermittelt werden kann. Ersichtlich ist nun eine Unterscheidung zwischen kalzifizierter Plaque und den Plaques niedrigerer Schwächung nicht mehr möglich, allerdings können lipidreiche Plaque und fibrotische Plaque, vgl. 3 und 4, sehr viel deutlicher getrennt werden.

Das Verfahren nutzt nun in einem gemeinsamen Workflow das Vorliegen der beiden Röntgenbilddatensätze 1 und 2 sowohl im Hinblick auf die Unterscheidungsmöglichkeiten in der Einenergie-Bildgebung als auch in der Multienergie-Bildgebung aus, wie im Folgenden beschrieben. Dabei ist zunächst ein optionaler Schritt S1 gezeigt, in dem der erste Röntgenbilddatensatz 1 und der zweite Röntgenbilddatensatz 2 miteinander registriert werden können, falls sie dies nicht ohnehin inhärent sind, beispielsweise aufgrund der Verwendung einer Dualenergie-Computertomographie-Einrichtung zur insbesondere gleichzeitigen Aufnahme. Es können übliche, automatisierte, elastische Registrierungsverfahren eingesetzt werden, um beide Röntgenbilddatensätze 1, 2 in ein gemeinsames Koordinatensystem zu überführen.

Die vorhandene Registrierung wird in einem Schritt S2 genutzt, um zum einen einen Mischbilddatensatz 6 und zum anderen einen Multienergiedatensatz 7 zu bestimmen. Dabei ergibt sich der Mischdatensatz 6 als eine voxelweise lineare Kombination der Röntgenbilddaten (HU-Werte) der Röntgenbilddatensätze 1, 2 derart, dass sie mit Gewichtungsparametern eingehen, die in der Summe 1 ergeben, beispielsweise mit p und (1-p), wobei p geeignet zwischen 0 und 1 zu wählen ist, beispielsweise als p = 0,5 für einen Durchschnittsdatensatz als Mischbilddatensatz 6. Der Mischbilddatensatz 6 entspricht also einem üblichen, bei einem bestimmten, festen Röntgenenergiespektrum aufgenommenen dreidimensionalen Bilddatensatz.

Der Multienergiedatensatz 7 wird durch voxelweise Berechnung des Dualenergieindex als Multienergiewert ermittelt; er enthält also für jedes Voxel den Multienergiewert.

In den Schritten S3 und S4 soll dann das interessierende Volumen lokalisiert werden, wofür vorliegend als Grundlage der Mischbilddatensatz 6 verwendet wird; es ist selbstverständlich genauso möglich den ersten Röntgenbilddatensatz 1, den zweiten Röntgenbilddatensatz 2 oder eine andere Kombination dieser beiden zu verwenden. Im Schritt S3 erfolgt unter Nutzung eines geeigneten Segmentierungsalgorithmus eine vollständig automatische Segmentierung des Blutgefäßbaums in dem von den Röntgenbilddatensätzen 1, 2 abgedeckten Koronarbereich des Patienten. Hierzu kann beispielsweise das im bereits erwähnten Artikel von Zheng et al. verwendete Verfahren eingesetzt werden.

Optional kann einem Benutzer das Segmentierungsergebnis in eine graphischen Benutzerschnittstelle angezeigt werden, damit dieser manuelle Nachkorrekturen vornehmen kann. In anderen Ausführungsbeispielen ist auch eine semiautomatische Segmentierung aufgrund von Eingaben eines Benutzers möglich.

In einem Schritt S4 wird dann auf Basis der Segmentierung eine Bildmaske definiert, die, angewandt auf die Datensätze 6, 7, jene Voxel selektiert, die im Hinblick auf die Gewebeverteilungsinformation im Folgenden genauer analysiert werden sollen. Dies kann vorliegend auf zwei unterschiedliche Arten geschehen. Zum einen ist es denkbar, dass der Benutzer auf assistierte Art und Weise einen Blutgefäßabschnitt wählt, der analysiert werden soll. Bevorzugt und im hiesigen Ausführungsbeispiel umgesetzt wird eine vollständig automatische Detektierung mittels geeigneter Softwarealgorithmen vorgenommen, die auch durch maschinelles Lernen parametriert sein können. Selbstverständlich kann auch in diesem Fall im Nachhinein eine manuelle Korrektur über eine entsprechende graphische Benutzerschnittstelle (GUI) erfolgen, wenn der Benutzer dies für nötig hält. Hierfür bekommt er die Lokalisierungsergebnisse angezeigt.

Fig. 5 zeigt beispielhaft einen Blutgefäßabschnitt 8 einer Koronararterie 9, in der eine Verengung durch eine Koronarplaque 10 vorliegt. Dabei können im Wesentlichen drei Gewebearten, wie oben bereits angedeutet, unterschieden werden. Vorliegend liegt ein nekrotischer Kern lipidreicher Plaque 11 unter einer dünnen Schicht ("Kappe") fibrotischer Plaque 12. In einem weiteren Bereich finden sich Einlagerungen in kalzifizierter Plaque 13.

Die zu bestimmende Bildmaske wird dabei idealerweise so bestimmt, dass sie exakt die Bildvoxel des Mischbilddatensatzes 6 und des Multienergiedatensatzes 7 angibt, für die die Gewebeart bestimmt werden soll, weswegen nicht nur aufgrund der vorangegangenen Segmentierungs- und Lokalisierungsergebnisse die gesunden Anteile der Gefäßwand ausgeschlossen werden können, sondern auch das Lumen 14, wozu beispielsweise eine semiautomatische oder bevorzugt automatische Segmentierung der inneren Gefäßwand stattfinden kann. Auf diese Weise wird die Trennung zwischen kalzifizierter Plaque 13 und kontrastverstärkt abgebildeten Blut im Gefäßlumen 14 verbessert.

Wurde die Bildmaske im Schritt S4, wie erwähnt bevorzugt möglichst weitgehend automatisch, definiert, kann zur Ermittlung der Gewebeverteilungsinformation fortgeschritten werden, die vorliegend die Anwendung zweier Klassifikatoren umfasst. Es sei angemerkt, dass selbstverständlich auch mehrere interessierende Bereiche definiert und entsprechend analysiert werden können.

In einem Schritt S5 wird zunächst ein erster Klassifikator auf den Mischbilddatensatz 6 angewendet, wobei im vorliegenden Ausführungsbeispiel ein Schwellwertvergleich auf die HU-Werte des Mischbilddatensatzes 6 angewandt wird, um zwischen zwei Gewebegruppen zu trennen. Die erste Gewebegruppe umfasst dabei lediglich kalzifizierte Plaque 13 bei hohen HU-Werten, die zweite Gewebegruppe jedoch zwei Gewebearten, nämlich lipidreiche Plaque 11 und fibrotische Plaque 12, die sich anhand der HU-Werte nicht sinnvoll unterscheiden ließen.

Der den ersten Klassifikator parametrierende HU-Schwellwert kann dabei aus einer Benutzereingabe heraus festgelegt werden, bevorzugt wird diese jedoch wenigstens mit einem automatisch bestimmten Vorschlagswert initiiert beziehungsweise der HU-Schwellwert wird vollständig automatisch bestimmt, indem Aufnahmeparameter und/oder Bildeigenschaften im interessierenden Bereich und/oder im Mischbilddatensatz insgesamt ausgewertet werden, insbesondere können auch hier Algorithmen der künstlichen Intelligenz eingesetzt werden, die durch maschinelles Lernen trainiert wurden. Grundsätzlich sind auch komplexere Klassifikatoren denkbar, was ebenso für den zweiten, noch zu diskutierenden Klassifikator gilt.

Ergebnis der Anwendung des ersten Klassifikators ist eine Zuordnung einer Gewebegruppe (und damit im Fall der ersten Gewebegruppe auch einer Gewebeart, hier der kalzifizierten Plaque 13) zu jedem durch die Bildmaske definierten Voxel.

Hierdurch wird mithin eine Auswahlmaske als weitere Bildmaske definiert, die es ermöglicht, Voxel, die der zweiten Gewebegruppe zugehörig sind und innerhalb der Bildmaske liegen, auszuwählen. Dies wird in einem zweiten Auswertungsschritt S6 ausgenutzt, indem ein zweiter Klassifikator auf die mittels der Auswahlmaske in den Multienergiedatensatz 7 selektierten Voxel angewandt wird, der den Dualenergieindex, der diesen Voxeln zugeordnet ist, voxelweise auswertet. Auch hier wird vorliegend wiederum ein Schwellwertvergleich eingesetzt, wobei der entsprechende DEI-Schwellwert wiederum benutzerseitig bestimmt sein kann, bevorzugt jedoch wieder unter Berücksichtigung der Aufnahmeparameter und/oder Bildeigenschaften wenigstens teilweise automatisch bestimmt wird. Auch hier sind selbstverständlich grundsätzlich komplexere zweite Klassifikatoren denkbar, insbesondere solche, die Algorithmen der künstlichen Intelligenz und Verfahren des maschinellen Lernens nutzen. Nachdem Voxel, die gemäß des ersten Klassifikators kalzifizierte Plaque 13 enthalten, ausgenommen sind, ist vgl. Fig. 4, eine Trennung zwischen lipidreicher Plaque 11 und fibrotischer Plaque 12 möglich. Ergebnis des zweiten Klassifikators ist also eine Zuordnung der durch die Auswahlmaske selektierbaren Voxel zu einer dieser beiden Gewebearten.

Somit ist für jedes Voxel, das durch die Bildmaske selektiert wurde, nun eine Gewebeart bestimmt worden, sodass diese Zuordnung mithin eine Gewebeverteilungsinformation bildet. Diese kann selbstverständlich noch weiter verfeinert werden, beispielsweise im Sinne einer quantitativen Gewebeverteilungsanalyse, für die beispielsweise den jeweiligen Gewebearten zugeordneten Voxel gezählt werden können. Auch innerhalb von Bereichen einer Gewebeart können weitere Analysen vorgenommen werden, beispielsweise Histogramme der dort vorhandenen Gewebearten/HU-Werte gemäß dem Mischbilddatensatz 6 zur weiteren Auswertung gebildet werden und dergleichen.

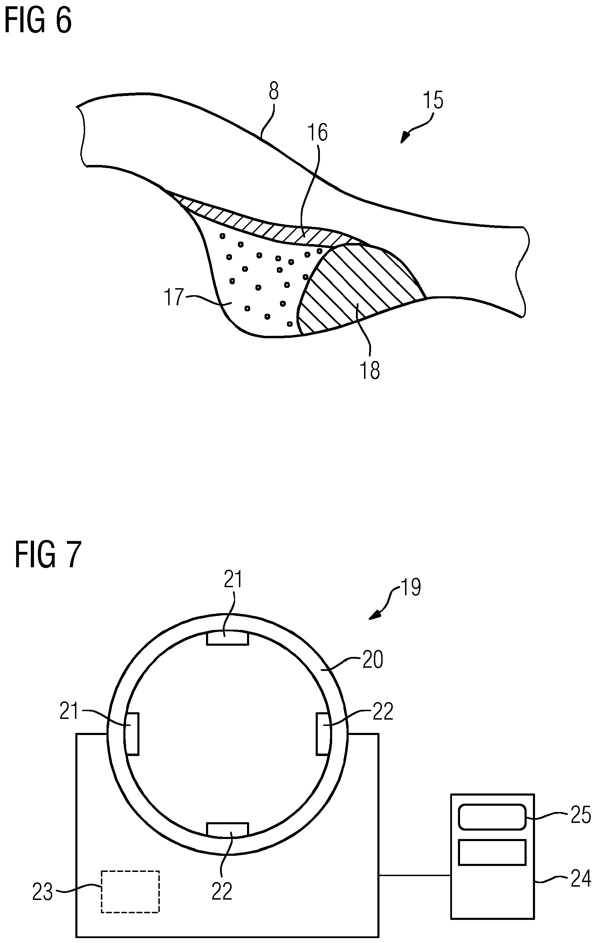

Eine entsprechende Ausgabe bestimmter Gewebeverteilungsinformationen erfolgt dann im Schritt S7, wobei Fig. 6 eine Möglichkeit zur Ausgabe der Gewebeverteilungsinformation zeigt. In der dortigen Darstellung 15 ist schematisch der Gefäßabschnitt 8 angedeutet, dem überlagert in verschiedenen Farben Bereiche 16, 17 und 18 dargestellt werden, die den verschiedenen Gewebearten zugeordnet sind. Mit der Vielzahl von Möglichkeiten konkreter Gewebeverteilungsinformation ergeben sich selbstverständlich auch verschiedenste weitere Optionen, Darstellungen zu erzeugen.

Fig. 7 zeigt schließlich eine Prinzipskizze einer erfindungsgemäßen Röntgeneinrichtung 19, die vorliegend als Dualenergie-Computertomographie-Einrichtung ausgebildet ist. Sie umfasst innerhalb der angedeuteten Gantry 20 zwei Aufnahmeanordnungen aus jeweils einem Röntgenstrahler 21 und einem Röntgendetektor 22, die mit verschiedenen Energieparametern, beispielsweise verschiedenen Röhrenspannungen, betrieben werden können, um die Röntgenbilddatensätze 1, 2 beziehungsweise die zugrundeliegenden zweidimensionalen Projektionsbilder aufzunehmen, aus denen dann, beispielsweise über gefilterte Rückprojektion, die dreidimensionalen Röntgenbilddatensätze 1, 2 rekonstruiert werden können.

Der Betrieb der Röntgeneinrichtung 19 wird mittels einer Steuereinrichtung 23 gesteuert, die auch zur Durchführung des erfindungsgemäßen Verfahrens ausgebildet ist. Um Bedieneingaben entgegenzunehmen und Informationen auszugeben, kann die Steuereinrichtung 23 hierbei auf eine Bedieneinrichtung 24 zugreifen, die auch eine entsprechende Anzeigevorrichtung 25, beispielsweise zur Ausgabe einer graphischen Nutzerschnittstelle, aufweist.

Zur konkreten Umsetzung des erfindungsgemäßen Verfahrens weist die Steuereinrichtung 23 verschiedene Subeinheiten auf, die durch Software und/oder Hardware realisiert sein können. Diese können beispielsweise eine Rekonstruktionseinheit zur Rekonstruktion der Röntgenbilddatensätze 1, 2, optional eine Registrierungseinheit, eine Ermittlungseinheit für den Mischbilddatensatz 6 und den Multienergiedatensatz 7, eine Segmentierungseinheit zur Durchführung des Schrittes S3, eine Lokalisierungseinheit zur Durchführung des Schrittes S4, eine erste Klassifikatoreinheit zur Durchführung des Schrittes S5, eine zweite Klassifikatoreinheit zur Durchführung des Schrittes S6 sowie zumindest wenigstens eine Interaktionseinheit zur Interaktion mit dem Benutzer, beispielsweise zur Durchführung des Schrittes S7, umfassen.

Zentral gesteuert werden kann der Verfahrensablauf auf der Steuereinrichtung 23 insbesondere durch wenigstens einen Prozessor.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden. Der Schutzumfang der Erfindung wird durch die angefügten unabhängigen Ansprüche definiert. Weitere Ausführungsbeispiele werden durch die abhängigen Ansprüche beschrieben.

## Patentansprüche

1. Verfahren zur Ermittlung einer Gewebeverteilungsinformation in einem interessierenden Bereich aus wenigstens einem dreidimensionalen Röntgenbilddatensatz (1, 2) eines Patienten, aufweisend folgende Schritte:
- Ermitteln von wenigstens zwei Röntgenbilddatensätzen (1, 2) aus bei unterschiedlichen, das Röntgenenergiespektrum beschreibenden Energieparametern aufgenommenen Röntgendaten,
- Bestimmen einer auf den durch die Röntgenbilddatensätze abgedeckten Raum anzuwendenden Bildmaske zur Lokalisierung des interessierenden Bereichs,
wonach zur Zuordnung von Gewebearten zu Voxeln im interessierenden Bereich als Gewebeverteilungsinformation:
- in einem ersten Zuordnungsschritt ein erster, wenigstens zwei Gewebegruppen mit wenigstens einer Gewebeart, von denen wenigstens eine Gewebegruppe wenigstens zwei Gewebearten umfasst, unterscheidender Klassifikator, der wenigstens einen der Röntgenbilddatensätze (1, 2) bezüglich eines Röntgenenergiespektrums und/oder einer linearen Kombination von Röntgenbilddaten unterschiedlicher Röntgenenergiespektren auswertet, auf den interessierenden Bereich angewandt wird, wobei aus dem Ergebnis der Anwendung des ersten Klassifikators für jede mittels wenigstens eines zweiten Klassifikators zu untersuchende Gewebegruppe eine Auswahlmaske bestimmt wird, die die Bildmaske weiter auf jene Voxel einschränkt, die einer durch den wenigstens einen zweiten Klassifikator weiter zu untersuchenden, wenigstens zwei Gewebearten enthaltenden Gewebegruppe zugeordnet sind, und
- in einem zweiten Zuordnungsschritt der wenigstens eine zweite, Gewebearten der wenigstens einen weiter zu untersuchenden, wenigstens zwei Gewebearten enthaltenden Gewebegruppe unterscheidender Klassifikator, der wenigstens einen aus wenigstens zwei der Röntgenbilddatensätze (1, 2) ermittelten, eine nichtlineare Kombination von Röntgenbilddaten unterschiedlicher Röntgenenergiespektren enthaltenden Multienergiewert auswertet, auf wenigstens den als der entsprechenden Gewebegruppe zugehörig klassifizierten Anteil des interessierenden Bereichs angewandt wird.

2. Verfahren nach Anspruch 1, **dadurch gekennzeichnet, dass** Röntgenbilddatensätze (1, 2) der Multienergie-Computertomographie, insbesondere der Dualenergie-Computertomographie, verwendet werden.

3. Verfahren nach Anspruch 1 oder 2, **dadurch gekennzeichnet, dass** als interessierender Bereich ein eine Koronarplaque enthaltender Gefäßabschnitt (8) eines Blutgefäßsystems des Patienten verwendet wird, wobei eine erste Gewebegruppe als einzige Gewebeart kalzifizierte Plaque (13) und eine zweite Gewebegruppe als Gewebearten lipidreiche Plaque (11) und fibrotische Plaque (12) umfassen.

4. Verfahren nach einem der vorangehenden Ansprüche, **dadurch gekennzeichnet, dass** die Röntgenbilddatensätze (1, 2) insbesondere elastisch registriert werden oder sind und/oder der erste Klassifikator einen aus den Röntgenbilddatensätze (1, 2)n durch Linearkombination von Röntgenbilddaten jeweiliger Voxel ermittelten Mischbilddatensatz (6) auswertet und/oder der wenigstens eine Multienergiewert für die verschiedenen Voxel in einem Multienergiedatensatz (7) des interessierenden Bereichs ermittelt wird.

5. Verfahren nach einem der vorangehenden Ansprüche, **dadurch gekennzeichnet, dass** als Multienergiewert der Dualenergieindex ermittelt wird.

6. Verfahren nach einem der vorangehenden Ansprüche, **dadurch gekennzeichnet, dass** zur Ermittlung des interessierenden Bereichs in den Röntgenbilddatensätzen (1, 2) eine automatische anatomische Segmentierung, insbesondere eine Segmentierung eines Blutgefäßsystems, durchgeführt wird, auf deren Grundlage der interessierende Bereich lokalisiert wird.

7. Verfahren nach Anspruch 6, **dadurch gekennzeichnet, dass** der interessierende Bereich manuell durch einen Benutzer in einer aus den Segmentierungsergebnissen abgeleiteten Modelldarstellung der Anatomie lokalisiert wird und/oder eine automatische Detektion des interessierenden Bereichs unter Berücksichtigung der Segmentierung und wenigstens einer in den Röntgenbilddaten sichtbaren anatomischen Eigenschaft des interessierenden Bereichs erfolgt.

8. Verfahren nach Anspruch 6 oder 7, **dadurch gekennzeichnet, dass** zur Lokalisierung die auf den durch die Röntgenbilddatensätze (1, 2) abgedeckten Raum anzuwendende Bildmaske bestimmt wird.

9. Verfahren nach Anspruch 8, **dadurch gekennzeichnet, dass** bei einem auf ein Gefäßorgan bezogenen interessierenden Bereich das Lumen des Gefäßorgans segmentiert und von der Maske ausgenommen wird.

10. Verfahren nach einem der vorangehenden Ansprüche, **dadurch gekennzeichnet, dass** der erste und/oder der zweite Klassifikator wenigstens einen Schwellwertvergleich umfassen.

11. Verfahren nach einem der vorangehenden Ansprüche, **dadurch gekennzeichnet, dass** wenigstens ein den ersten und/oder den zweiten Klassifikator parametrierender Klassifikatorparameter, insbesondere ein Schwellwert, wenigstens teilweise manuell und/oder wenigstens teilweise automatisch festgelegt wird.

12. Verfahren nach Anspruch 11, **dadurch gekennzeichnet, dass** zur wenigstens teilweise automatischen Ermittlung wenigstens eines Klassifikatorparameters wenigstens ein Aufnahmeparameter und/oder wenigstens ein die Röntgenbilddaten beschreibender Bilddatensatzparameter ausgewertet werden und/oder ein, insbesondere durch maschinelles Lernen anhand von bezüglich der Gewebearten annotierten Trainingsdaten trainiertes, physikalisches Parametermodell, insbesondere der künstliches Intelligenz, zur Ermittlung wenigstens teilweise automatisch zu bestimmender Klassifikatorparameter verwendet wird.

13. Röntgeneinrichtung (19), insbesondere Multienergie-Computertomographieeinrichtung, aufweisend eine zur Durchführung des Verfahrens nach Anspruch 1 ausgebildete Steuereinrichtung (23).

14. Computerprogramm, das die Schritte des Verfahrens nach Anspruch 1 durchführt, wenn es auf einer Recheneinrichtung ausgeführt wird.

15. Elektronisch lesbarer Datenträger, auf dem ein Computerprogramm nach Anspruch 14 gespeichert ist.

## Claims

1. Method for determining tissue distribution information in a region of interest comprising at least one three-dimensional x-ray image data record (1, 2) of a patient, having the following steps:
- determining at least two x-ray image data records (1, 2) from x-ray data recorded with different energy parameters describing the x-ray energy spectrum,
- ascertaining an image mask to be applied to the space covered by the x-ray image data records in order to localise the region of interest,
whereby in order to assign tissue types to voxels in the region of interest as tissue distribution information:
- in a first assignment step a first classifier, which differentiates at least two tissue groups with at least one tissue type, of which at least one tissue group comprises at least two tissue types, and which evaluates at least one of the x-ray image data records (1, 2) with respect to an x-ray energy spectrum and/or a linear combination of x-ray image data of different x-ray energy spectra, is applied to the region of interest, wherein a selection mask is determined from the result of the application of the first classifier for each tissue group to be examined by means of at least one second classifier, said selection mask further limiting the image mask to those voxels which are assigned to a tissue group to be examined further by the at least one second classifier and containing at least two tissue types, and
- in a second assignment step the at least one second classifier which differentiates tissues types of the at least one tissue group to be examined further and containing at least two tissue types and which evaluates at least one multi-energy value determined from at least two of the x-ray image data records (1, 2) and containing a non-linear combination of x-ray image data of different x-ray energy spectra is applied to at least the classified part of the region of interest which belongs to the corresponding tissue group.

2. Method according to claim 1, **characterised in that** x-ray image data records (1, 2) of the multi-energy computed tomography, in particular the dual energy computed tomography, are used.

3. Method according to claim 1 or 2, **characterised in that** a vascular segment (8) of a blood vessel system of the patient which contains a coronary plaque is used as the region of interest, wherein a first tissue group comprises calcified plaque (13) as a sole tissue type and a second tissue group comprises lipid-rich plaque (11) and fibrotic plaque (21) as tissue types.

4. Method according to one of the preceding claims, **characterised in that** the x-ray image data records (1, 2) will be or are registered in particular elastically and/or the first classifier evaluates a mixed image data record (6) determined from the x-ray image data records (1, 2) by linearly combining x-ray image data of the respective voxel and/or the at least one multi-energy value is determined for the different voxels in a multi-energy data record (7) of the region of interest.

5. Method according to one of the preceding claims, **characterised in that** the dual-energy index is determined as the multi-energy value.

6. Method according to one of the preceding claims, **characterised in that** in order to determine the region of interest in the x-ray image data records (1, 2), an automatic anatomical segmentation, in particular a segmentation of a blood vessel system, is carried out, on the basis of which the region of interest is localised.

7. Method according to claim 6, **characterised in that** the region of interest is localised manually by a user in a model representation of the anatomy derived from the segmentation results, and/or an automatic detection of the region of interest is carried out by taking into consideration the segmentation and at least one anatomical property of the region of interest which is visible in the x-ray image data.

8. Method according to claim 6 or 7, **characterised in that** for localisation purposes the image mask to be applied to the room covered by the x-ray image data records (1, 2) is determined.

9. Method according to claim 8, **characterised in that** with a region of interest relating to a vascular organ, the lumen of the vascular organ is segmented and excluded from the mask.

10. Method according to one of the preceding claims, **characterised in that** the first and/or the second classifier comprise at least one threshold value comparison.

11. Method according to one of the preceding claims, **characterised in that** at least one classifier parameter parameterising the first and/or the second classifier, in particular a threshold value, is defined at least partially manually and/or at least partially automatically.

12. Method according to claim 11, **characterised in that** in order to at least partially automatically determine at least one classifier parameter, at least one recording parameter and/or at least one image data record parameter describing the x-ray image data are evaluated and/or a physical parameter model trained in particular by machine learning on the basis of training data annotated with respect to the tissue types, in particular the artificial intelligence, is used to determine classifier parameters to be determined at least partially automatically.

13. X-ray facility (19), in particular multi-energy computed tomography facility, having a control facility (23) embodied to carry out the method according to claim 1

14. Computer program which carries out the steps of the method according to claim 1, when it is executed on a computing facility.

15. Electronically readable data medium, on which a computer program according to claim 14 is stored.

## Revendications

1. Procédé de détermination d'une information de répartition de tissus dans une zone à laquelle on s'intéresse, à partir d'au moins un ensemble (1, 2) de données d'image aux rayons X en trois dimensions d'un patient, comportant les stades suivants :
- on détermine au moins deux ensembles (1, 2) de données d'image aux rayons X, à partir de données aux rayons X prises pour des paramètres d'énergie différents décrivant le spectre d'énergie des rayons X,
- on détermine, pour la localisation de la zone à laquelle on s'intéresse, un masque d'image à appliquer sur l'espace recouvert par les ensembles de données d'image aux rayons X,
dans lequel, pour l'association des types de tissu à des voxels dans la zone à laquelle on s'intéresse, comme information de répartition de tissus :
- dans un premier stade d'association, on applique, à la zone à laquelle on s'intéresse, un premier classificateur distinguant au moins deux groupes de tissus ayant au moins un type de tissu, dont au moins un groupe de tissus comprend au moins un type de tissus, classificateur, qui évalue au moins l'un des ensembles (1, 2) de données d'image aux rayons X, en ce qui concerne un spectre d'énergie de rayons X et/ou une combinaison linéaire de données d'image aux rayons X de spectres d'énergie de rayons X différents, dans lequel, à partir du résultat de l'application du premier classificateur, on détermine, pour chaque groupe de tissus à examiner, au moyen d'au moins un deuxième classificateur, un masque de sélection, qui limite le masque d'image davantage à chaque voxel, qui sont associés à un groupe de tissus contenant au moins un groupe de tissus à examiner davantage par le au moins un deuxième classificateur, et
- dans un deuxième stade d'association, le au moins un deuxième classificateur, qui distingue des types de tissus du au moins un groupe de tissus à examiner davantage et contenant au moins deux types de tissus et qui évalue une valeur de multi-énergies déterminée à partir d'au moins deux des ensembles (1, 2) de données d'image aux rayons X et contenant une combinaison non linéaire de données d'image aux rayons X de spectres d'énergie de rayons X différents, est appliqué au moins à la partie, classée comme appartenant aux groupes de tissus correspondant, de la zone à laquelle on s'intéresse.

2. Procédé suivant la revendication 1, **caractérisé en ce que** l'on utilise des ensembles (1, 2) de données d'image aux rayons X de la tomodensitométrie par ordinateur à multi-énergies, notamment de la tomodensitométrie par ordinateur à énergie duale.

3. Procédé suivant la revendication 1 ou 2, **caractérisé en ce que** l'on utilise, comme zone à laquelle on s'intéresse, un tronçon (8) de vaisseau, contenant une plaque coronaire, d'un système de vaisseaux sanguins du patient, dans lequel un premier groupe de tissus comprend une plaque (13) calcifiée comme seul type de tissu, et un deuxième groupe de tissus comprend, comme type de tissus, une plaque (11) riche en lipides et une plaque (12) fibrotique.

4. Procédé suivant l'une des revendications précédentes, **caractérisé en ce que** les ensembles (1, 2) de données d'image à rayons X sont enregistrées, en particulier élastiquement, et/ou le premier classificateur évalue un ensemble (6) de données d'image mélangé déterminé à partir des ensembles (1, 2) de données d'image aux rayons X, par combinaison linéaire de données d'image radiographiques de voxels respectifs, et/ou la au moins une valeur de multi-énergies est déterminée pour les divers voxels dans un ensemble (7) de données de multi-énergies de la zone à laquelle on s'intéresse.

5. Procédé suivant l'une des revendications précédentes, **caractérisé en ce que** l'on détermine, comme valeur de multi-énergies, l'indice d'énergie duale.

6. Procédé suivant l'une des revendications précédentes, **caractérisé en ce que**, pour la détermination de la zone à laquelle on s'intéresse, on effectue, dans l'ensemble (1, 2) de données d'image aux rayons X, une segmentation anatomique automatique, en particulier une segmentation d'un système de vaisseaux sanguins, sur la base de laquelle on localise la zone à laquelle on s'intéresse.

7. Procédé suivant la revendication 6, **caractérisé en ce que** l'on localise la zone à laquelle on s'intéresse manuellement par un utilisateur dans une représentation par modèle, déduite des résultats de la segmentation de l'anatomie, et/ou on effectue une détection automatique de la zone à laquelle on s'intéresse en tenant compte de la segmentation et d'au moins une propriété anatomique visible dans les données d'image aux rayons X.

8. Procédé suivant la revendication 6 ou 7, **caractérisé en ce que**, pour la localisation, on détermine le masque d'image à appliquer à l'espace recouvert par l'ensemble (1, 2) de données d'image aux rayons X.

9. Procédé suivant la revendication 8, **caractérisé en ce que** l'on segmente, pour une zone à laquelle on s'intéresse rapportée à un organe de vaisseaux, le lumen de l'organe de vaisseaux et on l'exclut du masque.

10. Procédé suivant l'une des revendications précédentes, **caractérisé en ce que** le premier et/ou le deuxième classificateurs comprend au moins une comparaison de valeurs de seuil.

11. Procédé suivant l'une des revendications précédentes, **caractérisé en ce que** l'on fixe au moins en partie manuellement et/ou au moins en partie automatiquement, au moins un paramètre de classificateur paramétrant le premier et/ou le deuxième classificateur, en particulier une valeur de seuil.

12. Procédé suivant la revendication 11, **caractérisé en ce que**, pour la détermination au moins en partie automatique, d'au moins un paramètre de classificateur, on évalue au moins un paramètre d'enregistrement et/ou au moins un paramètre d'ensemble de données d'image décrivant les données d'image aux rayons X, et/ou on utilise, pour la détermination de paramètres de classificateur à déterminer au moins en partie automatiquement, un modèle de paramètres physique, ayant subi un apprentissage, en particulier par apprentissage automatique, à l'aide de données d'apprentissage annotées concernant les types de tissus.

13. Dispositif (19) à rayons X, en particulier dispositif de tomodensitométrie assisté par ordinateur multi-énergies, comportant un dispositif (23) de commande constitué pour effectuer le procédé suivant la revendication 1.

14. Programme d'ordinateur, qui effectue les stades du procédé suivant la revendication 1, lorsqu'il est exécuté sur un dispositif informatique.

15. Support de données, déchiffrables électroniquement, sur lequel est mis en mémoire un programme d'ordinateur suivant la revendication 14.
